# EUROPÄISCHE PATENTSCHRIFT

(11) **EP 1 603 480 B1**
(45) Veröffentlichungstag und Bekanntmachung des Hinweises auf die Patenterteilung: **15.07.2009**
(21) Anmeldenummer: 03773417.5
(22) Anmeldetag: 02.12.2003
(51) Int. Cl.: A61C 9/00, A61K 6/00, A61K 6/10

(54) **DENTALSET FÜR DIE SULKUS-RETRAKTION**
DENTAL SET for SULCUS RETRACTION METHOD
ENSEMBLE DENTAIRE pour PROCEDE DE RETRACTION DU SILLON

(30) Priorität: 19.03.2003 EP 03006052
(43) Veröffentlichungstag der Anmeldung: 14.12.2005
(73) Patentinhaber: Coltène AG, 9450 Altstätten (SG) (CH)
(72) Erfinder: LÜBBERS, Dierk, 9450 Altstätten (CH); LAMPL, Stephan, CH-9450 Lüchingen (CH); KOLLEFRATH, Ralf, CH-9464 Rüthi (CH)
(74) Vertreter: Hepp, Dieter
(86) Internationale Anmeldenummer: PCT/CH2003/000798
(87) Internationale Veröffentlichungsnummer: WO 2004/082510

(56) Entgegenhaltungen:
- DE-A- 3 737 552
- US-A- 4 677 139
- US-A- 5 676 543
- US-B1- 6 398 761

## Beschreibung

Die Erfindung betrifft ein Dentalset zur Freilegung des Zahnhals-Bereich im Rahmen einer Abformung der Gebiss-Situation.

Für die Erstellung von Zahnersatz, insbesondere bei Zahn-Brücken muss dem Zahntechniker ein Abdruck der individuellen Situation der betroffenen Zähne bzw. des benachbarten Kieferabschnittes vorliegen. Hierzu wird deshalb vom Zahnarzt eine Abformung dieser Gebiss-Situation vorgenommen. Um den Übergang vom Zahnersatz zum Kiefer möglichst zu verdecken, ist es erforderlich, den Zahnhals freizulegen, welcher durch das Zahnfleisch verdeckt wird. Deshalb muss bei der Erstellung des Gebiss-Abdrucks dieser Bereich, d.h. der Sulkus freigelegt werden.

Neben einem weit verbreiteten Verfahren, bei dem ein sogenannter Retraktionsfaden in den Bereich des Sulkus eingebracht wird, beschreibt US 5,676,543 einen weiteren Weg. Gemäss dieser Schrift wird mit einer härtbaren Formmasse ein Abdruck der Gebiss-Situation erstellt. Die ausgehärtete Form wird nachfolgend entfernt. In diese Form wird eine Schicht eines spritzfähigen und härtbaren Materials aufgebracht und die so präparierte Form erneut in den Mund eingesetzt. Das spritzfähige und härtbare Material muss im weiteren zwingend eine blutstillende oder Gewebe-Kontraktion verursachende Verbindung enthalten. Durch Druckanwendung auf die Form wird dieses härtbare Material in den Bereich zwischen Zahnhals und Zahnfleisch gepresst. Durch das blutstillende Material wird eine Gewebekontraktion verursacht. Der Sulkus zieht sich vom Zahnhals zurück. Nach dem Säubern kann eine genaue Abformung der Zahnsituation einschliesslich des freigelegten Sulkus erfolgen.

Es hat sich allerdings herausgestellt, dass das Einbringen des spritzfähigen, härtbaren Materials in die negative Gebiss-Abformung nicht immer zu den gewünschten Resultaten führt. Teilweise wird beim Einsetzen der Abformung ein Teil des spritzfähigen Materials verschmiert, so dass eine korrekte Sulkus-Retraktion nicht mehr gewährleistet ist. Schliesslich ist auch die Kontraktion des Sulkus durch das blutstillende Mittel nicht immer ausreichend, um die Abdruckqualität zu gewährleisten. Im weiteren gestaltet sich auch die mengenmässige Applikation des spritzfähigen Materials schwierig, da beim Auftragen die Konturen der Negativ-Form verdeckt werden und somit keine Kontrolle der Schichtdicke mehr möglich ist.

US 4,677,139 beschreibt ein weiteres Verfahren Zur Sulkusretraktion under Verwendung eines expandierenden Materials und eines Trägers.

Die Aufgabe der Erfindung ist es, die Nachteile des Bekannten zu vermeiden, insbesondere eine alternative Methode zur Freilegung des Zahnhalses bereitzustellen.

Dies wird mit einen Dentalset gemäss dem unabhängigen Ansprüch gelöst.

Die Auwendung des Dendalsets weist eine Reihe von aufeinanderfolgenden Schritten auf. In den Grenzbereich zwischen Zahn und Zahnfleischrand wird mit einem geeigneten Instrument ein Silikonmaterial aufgetragen. Das kennzeichnende Merkmal dieses Silikonmaterials ist dessen Volumenexpansion bei der Aushärtung. Ein Kompressionshütchen wird aufgesetzt, wodurch dieses Kompressionshütchen, eine einseitige Begrenzung für die Expansion des Silikonmaterials bildet. Die Expansion des Silikons kann nur in Richtung des Sulkus erfolgen, so dass dieser vom Zahnhals abgelöst wird. Der

Sulkus wird also vom Zahnhals gelöst und nicht nur durch Gewebekontraktion vom Zahnhals zurückgezogen.

Für die Applikation des Silikonmaterials werden vorzugsweise dem Fachmann bekannte Mehrkammer-Kartuschensysteme, insbesondere Doppelkammer-Kartuschensysteme verwendet. Besonders bevorzugt sind diese Kartuschensysteme mit einer statischen Mischvorrichtung (bspw. Statomix von Mixpac, ConProTec Inc.) verbunden bzw. verbindbar, welche die in den Kartuschensystemen getrennt vorliegenden Komponenten vor dem Auftragen durchmischen. Derartige Kartuschensysteme sind bspw. von MIXPAC (ConProTec Inc.) in typischen Volumina von 5 ml, 10 ml und 50 ml erhältlich. Der Auslass ist üblicherweise mit einer Stahlhohlnadel bzw. einem Oraltip zum Auftragen der jeweiligen Mischung versehen.

Das bei der Aushärtung expandierende Silikonmaterial kann aus einer einzigen Silikonverbindung oder einem Gemisch verschiedener Silikonverbindungen bestehen. Bevorzugt weist dieses expandierende Silikonmaterial eine minimale Expansion von mindestens 20 %, weiter bevorzugt von mindestens von 35 %, besonders bevorzugt von mindestens 50 % und meist bevorzugt von mindestens 70 % bezogen auf das ursprüngliche Volumen auf. Die

Werte für diese Volumenexpansion beziehen sich hierbei auf die Expansion des Material in einem nicht begrenzten, d.h. offenen Volumen. Der Begriff expandierend ist im Rahmen der Erfindung so zu verstehen, dass die Volumenexpansion während oder nach der Härtungsreaktion des Silikonmaterials bzw. eines Gemisches aus mehreren Silikonverbindungen erfolgt. Als Härtungsreaktion sind Reaktionsabläufe zur verstehen, welche zur Ausbildung von neuen inter- oder intramolekularen Verknüpfungen führen. Die Expansion wird in der Regel verzögert nach dem Beginn dieser Reaktionsabläufe einsetzen und über das Ende dieser Reaktionen hinaus andauern. Geeignete Silikonverbindung, welche eine solches Expansionsverhalten zeigen, sind additionsvernetzende Silikonverbindungen. Ein expandierendes Silikonmaterial mit den zuvor angegebenen Expansionsvolumen erlaubt eine verbesserte und einfachere Steuerung der Sulkus-Retraktion. Gegebenenfalls wird vor der Auftragung des Silikonmaterials ein Härtungskatalysator zugemischt oder das Silikonmaterial enthält bereits einen Katalysator, der die Aushärtung aufgrund der vorhandenen Feuchtigkeit auslöst. Neben dem spezifischen Expansionsverhalten kann auch durch die aufgetragene Menge steuernd eingegriffen werden. Beide Faktoren beeinflussen daher gegenseitig das Ergebnis der Retraktion. Vorteilhaft ist ein additionsvernetzendes, expandierendes Silikonmaterial, welches als Zwei-Komponentensystem eingesetzt wird. Die als wesentlichen Bestandteile der beiden Komponenten verwendeten unterschiedlich funktionalisierten Poly(dimethyl)siloxane, beispielsweise Hydrogen-Dihydroxy- oder Divinyl-Poly(dimethyl)siloxane weisen eine Viskosität vorzugsweise zwischen 5 und 100 Pa.s auf. Die beiden Komponenten enthalten weiterhin Füllstoffe, die üblicherweise für Dentalmassen eingesetzt werden. Diese Füllstoffe können entweder oberflächenbehandelt oder ohne Oberflächen-Behandlung sein. Beispiele für Füllstoffe sind Kieselerde, pyrogene Kieselsäure, Calciumcarbonat, Quarzmehl oder Silicate.

Die Verwendung additionsvernetzender Silikone vermeidet mögliche gesundheitliche Beeinträchtigungen des Patienten, da bei der Aushärtung keine nachteiligen Verbindungen freigesetzt, d.h. abgespalten werden. Im weiteren besitzen diese Art von Verbindungen den Vorteil, dass sie sich nicht mit dem Abformungsmaterial verbinden. Nach der Aushärtung des expandierenden Silikons kann dieses entweder in einem Stück oder in wenigen Teilstücken vom Zahnbereich entfernt werden. Gleichzeitig entsteht für den Zahnarzt die Möglichkeit, den Erfolg der Retraktion zu kontrollieren, gegebenenfalls erneut expandierendes Silikonmaterial an die ungenügend freigelegte Stelle aufzubringen und den Retraktionsvorgang zu wiederholen. Diese Vorgehensweise ist mit einer Silikonverbindung, welche sich dauerhaft an die Abformung bindet, nicht möglich. Hier wäre eine vollständige Wiederholung der gesamten Prozedur erforderlich.

Weiter vorteilhaft ist das Verfahren, wenn vor der Auftragung des expandierenden Silikonmaterials auf den Grenzbereich zwischen Zahn und Zahnfleisch mindestens eine blutungsstillende Verbindung bzw. ein Adstringend aufgetragen wird. Beispielhafte blutungsstillende Verbindungen oder Adstringens sind einschliesslich der verschiedenen, geeigneten Hydrate Kaliumaluminiumsulfat, Aluminiumsulfat, Aluminiumeisensulfat, Aluminiumammoniumsulfat, Eisenchlorid, Aluminiumchlorid, Natriumchlorid, Zinkchlorid, Zinkphenolsulfat, Gerbsäuren, Adrenalin oder weitere bekannte Verbindungen. Durch die Auftragung dieser Verbindungen können bei der Expansion des Silikonmaterials entstehende Blutung unmittelbar zum Stehen gebracht werden. Somit lässt sich der verdrängte Sulkus nach Entfernen des ausgehärteten und expandierten Silikons einfach von ausgetretener Flüssigkeiten reinigen und bleibt für eine nachfolgende Detailabformung der Gebisssituation weitgehend frei von weiteren Flüssigkeitsaustritten. Da meist eine chemische Kompatibilität der blutungsstillenden Verbindung mit dem expandierenden Silikon nicht gewährleistet ist, müssen deshalb beide Komponenten getrennt aufgetragen werden. Einzelne blutstillende Verbindungen wie beispielsweise Gerbsäuren lassen sich allerdings in das additionsvernetzende, expandierende Silikonmaterial einarbeiten, so dass eine gemeinsame Auftragung auf den Bereich zwischen Zahn und Zahnfleisch möglich ist.

Zur Sulkus-Retraktion werden Röllchen verwendet, insbesondere Baumwollröllchen. Diese Röllchen können zylindrisch geformt sein; andere Formen können ggf. zweckmässig sein. Beispielsweise können die Kompressionshütchen Comprecap^{™} benützt werden. Die Röllchen werden dort angeordnet, wo der Sulkus freigelegt wird, nachdem das expandierende Silikonmaterial appliziert wurde. Die Baumwollröllchen wirken als Sperre für das expandierende Silikonmaterial.

In einer weiteren Ausführungsform wird das gehärtete, expandierte Silikonmaterial mit Hilfe eines Fadens entfernt, bspw. mit Hilfe eines Retraktionsfadens, welcher nach Applikation des Silikonmaterials in das expandierende Silikonmaterial eingelassen wird. Der Faden wird durch die Aushärtung des Silikonmaterials in diesem fixiert. Der Faden kann selbstverständlich ebenfalls im Bereich des Zahnhalses angeordnet und mit dem Silikonmaterial abgedeckt werden. Alle Materialien, welche für den Dentalbereich geeignet sind, können für einen solchen Faden verwendet werden.

Des ordindungssemässe Dentalset wird zur Ausführung des oben ausgeführten Verfahrens eingesetzt. Das Set umfasst ein Kompressionshütchen und mindestens ein expandierendes Silikonmaterial, wobei die Volumenexpansion mindestens 20 %, vorzugsweise mindestens 35 %, weiter bevorzugt 50 % und besonders bevorzugt 70 % gegenüber dem ursprünglichen Volumen der nicht ausgehärteten Mischung beträgt.

Weiter vorteilhaft enthält dieses Set eine blutungsstillende Verbindung analog den bereits vorstehend beschriebenen, welche separat auf die zu behandelnde Stelle aufzutragen ist.

In einer weiteren Ausführungsform eines erfindungsgemässen Sets beinhaltet das expandierende Silikonmaterial eine blutstillende Verbindung, vorzugsweise wenigstens eine Gerbsäure. Diese Mischung einer blutstillenden Verbindung, insbesondere mindestens einer Gerbsäure, und dem expandierenden Silikonmaterial wird in dem betreffenden Bereich appliziert.

Die Erfindung wird im folgenden anhand von Beispielen und der Figur näher erläutert.

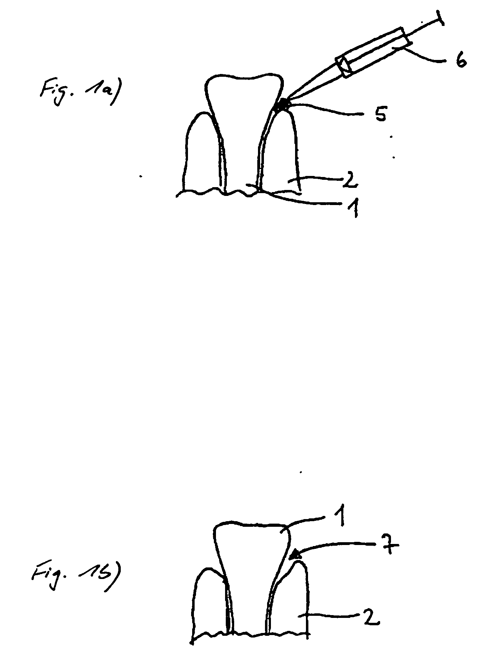

- Figuren 1a bis b:: Darstellung der wesentlichen Verfahrensschritte

### Beispiel 1:

Bevor im weiteren die expandierende Silikonverbindung aufgebracht wird, erfolgt ein Aufpinseln von Aluminiumsulfat als blutungsstillender Verbindung. In den Grenzbereich zwischen Zahn 1 und dem Gewebe 2 wird mit einem in-situ Mischapplikator 6 die expandierende Silikonverbindung 5 eingespritzt Fig. 1a. Diese expandierende Silikonverbindung 5 besteht aus zwei Komponenten A und B.

Die Komponente A setzt sich zusammen aus:

| | |
|---|---|
| 10g | alpha, omega-Dihydroxy-Polydimethylsiloxan (Viskosität 18 Pa.s; Wacker Silicones) |
| 5g | Quarzmehl Sikron B600 (Quarzwerke Frechen, D) |
| 0.05g | Silopren U-Katalysator Pt/D (GE Bayer Silicones) |
| 0.02g | Divinyltetramethyldisiloxan (Fluka) |

Die Komponente B weist folgende Zusammensetzung auf:

| | |
|---|---|
| 9.8g | alpha, omega-Divinyl-polydimethylsiloxan (Viskosität 20 Pa.s; Wacker Silicones) |
| 5g | Quarzmehl Sikron B600 (Quarzwerke Frechen, D) |
| 0.3g | Polymethylhydrosiloxan (Viskosität 20 mPa.s; Wacker Silicones) |

Beide Komponenten A und B werden im Masseverhältnis 1:1 homogen gemischt. Durch das unmittelbares Mischen der Silikonkomponenten A und B beim Auftragen wird die Aushärtung gestartet.

Die Begrenzung der Ausdehnung des expandierenden Silikon-Komponentengemisches 5 wird bewirkt durch Comprecap^{™} Kompressionshütchen (Coltène Whaledent/Roeko).

Die benötigte Anzahl Comprecaps^{™} wird in haltbarer Ausrichtung in jenen Bereichen angeordnet, wo der Sulkus vom Zahnhals entfernt werden muss. Die Comprecaps^{™} bedecken hierbei die Oberfläche des aufgetragenen, expandierenden Silikonmaterials, welche nicht in Kontakt mit dem Zahn oder dem Gingiva-Gewebe steht, also jene Oberfläche, welche von Zahn und Gingiva abgewendet ist. Nach Härtung und gleichzeitiger Ausdehnung des Silikonmaterials wird das Silikonmaterial gemeinsam mit den Comprecaps^{™} entfernt.

Die endgültige Abformung der Zahnsituation kann nach einer Reinigung des erweiterten Sulkus mit einer hohen Präzision vorgenommen werden. Figur 1b zeigt den Zahn 1 mit dem freigelegten Sulkus 7 und dem darunter liegenden Zahnfleisch 2.

In einer Variante dieses Verfahrens wird ein Retraktionsfaden Retracto^{™} (Coltene Whaledent/Roeko) in das Silikon-Komponentengemisch 5 nach dem Auftragen eingebracht. Mit Hilfe dieses Retraktionsfadens ist das gehärtete Silikonmaterial leicht entfernbar.

## Patentansprüche

1. Dentalset geeignet für eine Sulkus-Retraktion, enthaltend ein Kompressionshütchen und mindestens eine härtbare Silikonverbindung (5), wobei die Silikonverbindung (5) bei der Aushärtung eine Volumensexpansion von mindestens 20 %, vorzugsweise mindestens 35 % gegenüber dem ursprünglichen Volumen der nicht gehärteten Verbindung aufweist.

2. Dentalset nach Anspruch 1, **dadurch gekennzeichnet, dass** das Set eine blutungsstillende Verbindung enthält.

3. Dentalset nach Anspruch 1 oder 2, **dadurch gekennzeichnet, dass** die härtbare Abformmasse (3) mindestens eine weitere Silikonverbindung und einen Härtungskatalysator enthält.

4. Dentalset nach einem der Ansprüche 1 bis 3, **dadurch gekennzeichnet, dass** die härtbare und expandierende Silikonverbindung (5) aus der Gruppe der additionsvernetzenden Silikonverbindungen ausgewählt ist.

5. Dentalset nach einem der Ansprüche 1 bis 4, weiter beinhaltend mindestens ein Mehrkammer-Kartuschensystem, insbesondere mindestens ein Doppelkammer-Kartuschensystem, vorzugsweise verbunden bzw. verbindbar mit jeweils einer statischen Mischvorrichtung, zur Applikation der härtbaren Silikonverbindung (5).

6. Dentalset nach einem der Ansprüche 1 bis 5, Weiter enthaldend einen Retraktionsfaden.

7. Dentalset nach einem der Ansprüche 1 bis 6, wobei es sich bei dem Kompressionshütchen um ein Röllchen handelt, insbesondere ein Baumwollröllchen.

8. Dentalset nach einem der Ansprüche 1 bis 7, wobei das Röllchen Zylindrich ist.

## Claims

1. Dental set suitable for sulcus retraction, comprising a compression cap and at least one curable silicone compound (5), which silicone compound (5), during curing, undergoes a volume expansion of at least 20%, preferably of at least 35%, relative to the original volume of the uncured compound.

2. Dental set according to Claim 1, **characterized in that** the set comprises a haemostatic compound.

3. Dental set according to Claim 1 or 2, **characterized in that** the curable impression composition (3) comprises at least one further silicone compound and a curing catalyst.

4. Dental set according to one of Claims 1 to 3, **characterized in that** the curable and expanding silicone compound (5) is chosen from the group of addition-crosslinking silicone compounds.

5. Dental set according to one of Claims 1 to 4, further comprising at least one multi-chamber cartridge system, in particular at least one twin-chamber cartridge system, in each case preferably connected or connectable to a static mixer device, for applying the curable silicone compound (5).

6. Dental set according to one of Claims 1 to 5, further comprising a retraction thread.

7. Dental set according to one of Claims 1 to 6, in which the compression cap is a small roll, in particular a small roll of cotton.

8. Dental set according to one of Claims 1 to 7, in which the small roll is cylindrical.

## Revendications

1. Set dentaire convenant à une rétraction du sillon, contenant une coiffe de compression et au moins un composé siliconé durcissable (5), le composé siliconé (5) présentant, lors du durcissement, une dilatation volumique d'au moins 20 %, de préférence d'au moins 35 % par rapport au volume initial du composé non durci.

2. Set dentaire selon la revendication 1, **caractérisé en ce que** le set contient un composé hémostatique.

3. Set dentaire selon la revendication 1 ou 2, **caractérisé en ce que** la composition durcissable pour empreinte (3) contient au moins un composé siliconé supplémentaire et un catalyseur de durcissement.

4. Set dentaire selon l'une des revendications 1 à 3, **caractérisé en ce que** le composé siliconé durcissable et dilatable (5) est choisi dans le groupe des composés siliconés pouvant subir une réticulation par addition.

5. Set dentaire selon l'une des revendications 1 à 4, contenant en outre au moins un système de cartouche à plusieurs compartiments, en particulier au moins un système de cartouche à deux compartiments, de préférence reliés ou pouvant être reliés chacun à un dispositif mélangeur statique, pour application du composé siliconé durcissable (5).

6. Set dentaire selon l'une des revendications 1 à 5, contenant en outre un fil de rétraction.

7. Set dentaire selon l'une des revendications 1 à 6, dans lequel, pour ce qui concerne la coiffe de compression, il s'agit d'un rouleau, en particulier d'un rouleau de coton.

8. Set dentaire selon l'une des revendications 1 à 7, dans lequel le rouleau est cylindrique.
